**Europäisches Patentamt**

**European Patent Office**

**Office européen des brevets**

(19)

(11) Veröffentlichungsnummer: **0 377 883**
**A1**

## EUROPÄISCHE PATENTANMELDUNG

(12)

(21) Anmeldenummer: **89123750.5**

(22) Anmeldetag: **22.12.89**

(51) Int. Cl.⁵: **C07H 15/04**

(30) Priorität: **11.01.89 DE 3900590**

(43) Veröffentlichungstag der Anmeldung:
**18.07.90 Patentblatt 90/29**

(84) Benannte Vertragsstaaten:
**GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Rossmaier, Henry, Dr.**
**Tannenhofweg 43**
**D-4000 Düsseldorf 12(DE)**
Erfinder: **Biermann, Manfred, Dr.**
**Markscheiderhof 25**
**D-4330 Mülheim/Ruhr(DE)**

(54) **Verfahren zur Herstellung von Alkylglucosiden.**

(57) Bei dem Verfahren nach der Umacetalisierungsreaktion mit Propylenglykol wird zunächst zu einem auf 100 bis 130 °C erwärmten Reaktionsmedium (A), das mindestens aus Propylenglykol und einem sauren Katalysator und gegebenenfalls höherem ($C_8$-$C_{18}$-)Fettalkohol besteht, eine Saccharid-Komponente (B) zugegeben, die Stärke oder partielle Stärkeabbauprodukte enthält, wobei zunächst Propylenglykol-Glucosid entsteht, das mit Fettalkohol umacetalisiert wird. Das molare Verhältnis von Fettalkohol zu Propylenglykol beträgt maximal 1. Nach an sich üblicher Aufarbeitung wird ein im Alkalischen farbstabiles Produkt mit hohem Anteil an $C_8$-$C_{18}$-Alkylmonoglucosid erhalten. Durch Destillation zurückgewonnene Mengen an Propylenglykol und Fettalkohol lassen sich ohne weiteren Aufbereitungsschritt recyclisieren.

EP 0 377 883 A1

## Verfahren zur Herstellung von Alkylglucosiden

Die Erfindung betrifft ein neues, verbessertes Verfahren zur Herstellung von Alkylglucosiden nach der Umacetalisierungsmethode mit Propylenglykol.

In zunehmendem Maße werden zur Entwicklung und Herstellung neuer oberflächenaktiver Stoffe, die als industrielle Tenside für die Herstellung von Wasch- und Reinigungsmitteln geeignet sind, nachwachsende Rohstoffe verwendet. Dafür kommen bisher hauptsächlich fettchemische Rohstoffe, wie z. B. Fettsäuren, Fettsäureester und Fettalkohole in Betracht. Unter diesen Gesichtspunkten sind neuerdings die oberflächenaktiven Alkylglucoside, bei denen es sich um Acetale aus Glucose und Fettalkoholen handelt, interessant geworden. Der Begriff Alkylglucosid soll die Alkylmonoglucoside und auch die Alkyloligo- bzw. Alkylpolyglucoside, insbesondere aber Gemische aus den Mono- und Oligoglucosiden, umfassen.

Der Begriff "Alkyl" in Alkylglucosid bezieht sich auf den Rest der entsprechenden primären Alkohole natürlichen und/oder synthetischen Ursprungs mit vorzugsweise $C_8$- bis $C_{18}$-Alkyl- bzw. Alkenylresten. Das für die Praxis wichtigste Saccharid-Einsatzmaterial sind in der Natur vielfältig zur Verfügung stehende wasserfreie Polyglucoseverbindungen, deren einzelne Glucoseeinheiten in $\alpha$-glucosidischer Verknüpfung vorliegen. Das wichtigste Einsatzmaterial natürlichen Ursprungs dieser Art ist die Stärke, die weltweit durch Nutzpflanzen unterschiedlichster Art, beispielsweise Kartoffeln, Mais, Tapioka, Reis und dergleichen, gebildet wird. Pulverförmige Stärken und ihre partiellen Abbau produkte, beispielsweise in Form eines entsprechenden, meist hochkonzentrierten Glucosesirups, stehen als vergleichsweise preiswerte Einsatzmaterialien zur Verfügung.

Aus den 60er und 70er Jahren stammt eine Vielzahl an Vorschlägen zu Herstellungsverfahren für Alkylglucoside, die sich insbesondere mit zwei Hilfselementen befassen. Zunächst einmal wurde auf Grund der hohen Empfindlichkeit der Polysaccharide gegenüber den bei der Herstellung der Alkylglucoside bisher benötigten hohen Temperaturen und Drücken vorgeschlagen, nicht die Polyanhydroglucosekomponenten als solche einzusetzen, sondern dieses Einsatzmaterial natürlichen Ursprungs zu Monosaccharid, d. h. zu Glucose abzubauen. Die Glucose kann dann als wasserfreies Material oder als Glucosehydrat der Acetalisierung zugeführt werden.

Das zweite eingesetzte Hilfsmittel benutzt die Tatsache, daß niedere Alkohole und Glykole, insbesondere solche mit einer Alkylkettenlänge im Bereich von $C_3$-$C_5$, vergleichsweise einfach zur gewünschten Acetalisierung am Monosaccharid führen. Die so gebildeten Acetale des Monosaccharids besitzen allerdings nur unzureichende Tensideigenschaften. Sie werden durch Umacetalisierung mit den längerkettigen monofunktionellen Alkoholen des Bereichs von $C_8$ bis $C_{18}$ in die gewünschten Alkylglucosidreaktionsprodukte mit oberflächenaktiven Eigenschaften umgewandelt. Auch dieser scheinbar vergleichsweise einfache Herstellungsweg ist für die Praxis mit zahlreichen einschränkenden Schwierigkeiten wie die Gewinnung hellfarbiger, farbstabiler und insbesondere im Alkalischen farbstabiler Alkylglucoside verbunden.

So beschreibt die europäische Patentanmeldung 102 558 (konkordant mit US 4 704 453) die Herstellung von langkettigen Alkylglucosid gemischen mittels einer Umacetalisierung von $C_3$-$C_5$-Alkylglucosiden. Das Verfahren geht von Glucose aus. Die europäische Patentanmeldung 99 183 beschreibt die Umwandlung von Sacchariden, insbesondere Polysacchariden wie Stärke, mit Alkoholen mit wenigstens 3 C-Atomen zu Glucosidgemischen. Hierbei soll die Umsetzung in Gegenwart von wenigstens 2 Mol Wasser je molare Saccharideinheit in Gegenwart von insbesondere sauren Katalysatoren durchgeführt werden. Bevorzugt enthält die zur Dispergierung der Polysaccharidverbindung eingesetzte Flüssigphase zusätzlich ein alkohollösliches organisches Hilfslösungsmittel. Als besonders geeignete Alkohole zur Bildung der Alkylglucosidverbindungen haben sich Alkanole mit 3 bis 6 C-Atomen und insbesondere mit 3 oder 4 C-Atomen herausgestellt. Die auf diese Weise gebildeten niederen Alkylglucoside können nach den Angaben dieser Druckschrift als Zwischenverbindungen bei der Herstellung von oberflächenaktiven Alkylglucosidverbindungen eingesetzt werden, ohne daß jedoch nähere Einzelheiten zu diesem nachfolgenden Reaktionsschritt angegeben sind.

Die US-amerikanische Patentschrift 3 772 269 beschreibt die Herstellung von Alkylglucosiden aus einer Saccharid-Komponente und längerkettigen monofunktionellen Alkoholen mit im wesentlichen $C_8$- bis $C_{25}$-Alkyl- bzw. Alkenylketten nach dem Umacetalisierungsverfahren mit aliphatischen $C_3$- bis $C_5$-Glykolen, wobei vorzugsweise Propylenglykol verwendet wird. Dabei werden in einem ersten Schritt Glykol, höherer Alkohol, der eine primäre oder sekundäre OH-Gruppe besitzt, Saccharid, wobei bevorzugt Glucose, aber auch Oligo- bzw. Polysaccharide eingesetzt werden, und ein saurer Katalysator miteinander vermischt. Anschließend wird diese Mischung auf die Reaktionstemperatur erwärmt, die in Abhängigkeit von den eingesetzten Komponenten zwischen 70 und 160 °C liegt. Die Aufarbeitung erfolgt in an sich bekannter Weise. Die erhaltenen Produkte zeichnen sich, je nach Verfahrensführung, durch einen hohen Anteil an Glykolglucosiden oder an Fettalkoholglucosiden aus. Um zu Produkten mit einem hohen Anteil an Fettalkoholglucosiden zu gelangen, bedarf es allerdings generell eines hohen Überschusses an Fettalkohol gegenüber eingesetztem Glykol. Über die Qualität der Produkte bezüglich Farb- und Alkalistabilität wird keine Aussage gemacht.

Die Erfindung geht von der Aufgabe aus, die als natürliche Einsatzmaterialien zur Verfügung stehenden Polysaccharide von der Art der Stärken oder ihrer partiellen Abbauprodukte derart in Alkylglucosidverbindungen mit oberflächenaktiven Eigenschaften umzuwandeln, daß es keiner Isolierung irgendwelcher Zwischenstufen bedarf und die Reaktionsprodukte dem geforderten Standard an Helligkeit, Farbstabilität und insbesondere Alkalistabilität genügen.

Gegenstand der Erfindung ist dementsprechend ein Verfahren zur Herstellung von Alkylglucosiden aus einer Saccharid-Komponente und längerkettigen monofunktionellen Alkoholen mit im wesentlichen $C_8$- bis $C_{18}$-Alkyl- bzw. Alkenylketten nach der Umacetalisierungsmethode mit Propylenglykol, dadurch gekennzeichnet, daß zu einem auf 100 bis 130 °C erwärmten Reaktionsmedium (A), das mindestens aus Propylenglykol und einem sauren Katalysator und gegebenenfalls höherem Fettalkohol besteht, eine Saccharid-Komponente (B), die Stärke oder partielle Stärkeabbauprodukte enthält, zudosiert wird, wobei der eingesetzte Alkohol eine primäre OH-Gruppe besitzt und das molare Verhältnis von höherem Alkohol zu Propylenglykol maximal 1 ist.

Als Glucoseeinsatzmaterial sind insbesondere Polysaccharide geeignet. Das bevorzugte Polysaccharid für die Verarbeitung im erfindungsgemäßen Verfahren ist Stärke beliebigen Ursprungs und/oder ihre partiellen Abbauprodukte, wie sie in Form meist hochkonzentrierter wäßriger sirupartiger Produkte zur Verfügung stehen.

Als Alkohole werden monofunktionelle aliphatische Alkohole, insbesondere primäre Alkohole einer C-Kettenzahl von 8 bis 18, verwendet. Bevorzugt sind lineare Alkohole natürlichen Ursprungs (Fettalkohole), aber auch synthetische primäre Alkohole, wie z.B. die sogenannten Oxoalkohole, die einen gewissen Prozentsatz, meist 20 bis 40 %, an verzweigten Isomeren mit einem 2-Methyl-Rest aufweisen, sind geeignet. Typische geeignete Alkohole sind demnach Decanol, Dodecanol, Tetradecanol, Hexadecanol, Octadecanol und 2-Methylundecanol sowie Mischungen der $C_{10}$-, $C_{12}$- und $C_{14}$-Alkohole.

Als saurer Katalysator wird vorzugsweise wegen ihrer im Vergleich zu Schwefelsäure geringeren korrodierenden Wirkung gegenüber Geräten und Leitungen aus Stahl die Paratoluolsulfonsäure verwendet. Prinzipiell sind aber als Katalysatoren alle sauren Verbindungen, einschließlich der Phosphorsäuren und der sogenannten Lewis-Säuren, welche die Acetalisierungsreaktion zwischen Fettalkohol und Zuckermolekül katalysieren, geeignet.

In einer bevorzugten Ausführungsform des Verfahrens wird ein Reaktionsmedium (A), das aus Propylenglykol in Mengen von 3 bis 8 Mol, bezogen auf 1 Mol Saccharid (berechnet als Anhydroglucose), und einem sauren Katalysator in einer Menge von 0,01 bis 0,03 Mol pro Mol der im eingesetzten Saccharid enthaltenen Glucose-Einheit besteht, auf die Reaktionstemperatur von 100 bis 130 °C erwärmt. Zu dieser vorgewärmten Mischung (A) wird anschließend die Saccharid-Komponente (B), die aus Stärke oder partiellen Stärkeabbauprodukten besteht, derart zudosiert, daß die Reaktionsmischung stets eine klare Lösung bleibt. Als verfahrenstechnisch günstig hat es sich erwiesen, eine Saccharid-Komponente (B) zuzudosieren, in der der Zucker in Propylenglykol suspendiert vorliegt. Vorzugsweise werden dabei Mengen von 2 bis 4 Mol Propylenglykol, bezogen auf 1 Mol Anhydroglucose, verwendet. Die Zugabe der Saccharid-Komponente (B) kann sowohl portionsweise als auch kontinuierlich erfolgen. Dabei ist die kontinuierliche Zugabe die bevorzugte Ausführungsform.

Das als Zwischenprodukt erhaltene Propylenglykol-Glucosid braucht nicht isoliert zu werden, da die Entfernung von Wasser und überschüssigem Propylenglykol unter reduziertem Druck bei etwa 100 mm Hg bei gleichzeitiger Eintragung des vorzugsweise auf 90 bis 100 °C vorgewärmten höheren Fettalkohols geschieht. Der Fettalkohol wird in Mengen von 3 bis 8 Mol pro Mol Anhydroglucose eingesetzt, wobei das molare Verhältnis von Fettalkohol zu Propylenglykol maximal 1 ist. In einer bevorzugten Ausführungsform wird der zu der durch Mischung von (A) und (B) entstandenen Reaktionsmischung zuzugebende Fettalkohol mit 0,5 bis 1 Mol Propylenglykol pro Mol eingesetztem Fettalkohol hydrophilisiert, wodurch ein synchroner Propylenglykol-Fettalkohol-Austausch erleichtert wird.

In einer weiteren bevorzugten erfindungsgemäßen Verfahrensführung wird als Reaktionsmedium (A) eine Mischung eingesetzt, die aus Propylenglykol und dem sauren Katalysator in den oben angegebenen Mengen und zusätzlich aus höherem Fettalkohol in Mengen von 3 bis 8 Mol pro Mol Anhydroglucose besteht. Die Zugabe der Saccharid-Komponente (B) zu dem erwärmten Reaktionsmedium (A) erfolgt wie oben beschrieben. Anschließend können einerseits Wasser und andererseits Propylenglykol durch stufenweise Reduktion des Druckes im Bereich von 100 bis 15 mm Hg bei einer Temperatur von 115 bis 120 °C separat destillativ entfernt werden. Das zurückgewonnene Propylenglykol, das auch Anteile an Fettalkohol

3

enthalten kann, läßt sich somit recyclisieren.

In einer weiteren bevorzugten Ausführungsform des erfindungsgemaßen Verfahrens wird dementsprechend ein Reaktionsmedium (A) verwendet, das aus Propylenglykol und Katalysator in den angegebenen Mengen, jedoch nur aus einem Teil des insgesamt zuzugebenden Fettalkohols besteht. Die restliche Menge Fettalkohol wird dann wie oben beschrieben in die intermediäre Propylenglykol-Glucosid-Mischung eingetragen.

Das nach dem erfindungsgemäßen Verfahren anfallende Endprodukt wird in an sich bekannter Weise gereinigt und aufgearbeitet, wie es im einzelnen in der eingangs geschilderten Literatur und in der deutschen Patentanmeldung 37 23 826 geschildert ist.

Die erfindungsgemäße Verfahrensführung sieht dabei vor, daß vor der Neutralisation des sauer katalysierten Reaktionsproduktes eine Filtration und damit eine Abtrennung des nicht umgesetzten Saccharids durchgeführt werden kann, das dann in einem weiteren Ansatz erneut als Ausgangsmaterial dient.

Zur Neutralisation des sauren Katalysators sind vor allem organische oder anorganische basische Alkali- oder insbesondere Erdalkaliverbindungen, vorzugsweise Organomagnesiumverbindungen wie Magnesiumalkoholate oder anorganische Magnesiumverbindungen wie Magnesiumoxid bzw. Magnesiumhydroxid, geeignet. Zweckmäßigerweise werden dabei pH-Werte von wenigstens 8, vorzugsweise von etwa 9 bis 10 eingestellt. Dabei stellt die Einstellung dieser Werte die Farbstabilität des Tensids im alkalischen Milieu bei seiner Lagerung sowie insbesondere bei seiner späteren Verarbeitung sicher.

Nach einer - weiteren - Filtration kann die Abdestillation des Fettalkoholüberschusses in bekannten produktschonenden Vakuumdestillationsvorrichtungen erfolgen. Geeignet ist hierfür insbesondere der Einsatz von Dünnschicht- und/oder Fallfilmverdampfern. Der zurückgewonnene Fettalkohol kann ebenfalls dem Verfahren erneut zugeführt werden.

Das Reaktionsendprodukt bildet im erkalteten Zustand eine schwach gelbliche wachsartige Masse, die vorzugsweise wegen der besseren Handhabbarkeit in eine wäßrige Paste mit ca. 60 % Wirkstoffgehalt übergeführt werden kann. Falls besonders hohe Ansprüche an die Farblosigkeit des Endproduktes gestellt werden, kann gleichzeitig mit der Herstellung der wäßrigen Paste eine Bleiche mit Wasserstoffperoxid oder einer organischen Persäure bzw. entsprechenden Persäuresalzen eine zusätzliche Farbkorrektur und Farbaufhellung erfolgen. Als zweckmäßig hat sich ein weiterer Zusatz in Form von Natriumhydroxid oder eines Natriumhydroxid/Citronensäure-Puffers erwiesen.

Die Farbstabilität des Produkts wird durch einen einfachen Test festgestellt. Dazu wird eine Probenmenge des Produkts mit Wasser zu einer ca. 50%igen Paste vermischt und bei Normaltemperatur mit konzentrierter Natronlauge versetzt, so daß sich ein pH-Wert von ca. 12 bis 13 einstellt. Dann wird 60 Minuten lang auf 100 °C erhitzt. Bei den Ansätzen mit Verfahrensprodukten trat nach dieser Behandlung keine oder keine wesentliche Farbänderung ein. Die Farbzahlen für die Produkte wurden nach der Methode von KLETT bestimmt (5 %ige Lösung in Wasser/Isopropanol 70 : 25, 1 cm-Küvette, Blaufilter). Mit dieser Testmethode können Langzeitlagerversuche des Produkts unter üblichen Bedingungen sowie Weiterverarbeitungsverfahren des gelagerten Produkts zu insbesondere Wasch- und Reinigungsmitteln und den dabei auftretenden alkalischen Bedingungen zuverlässig simuliert werden. Die Verfahrensprodukte besitzen vorzugsweise Klett-Zahlen von weniger als 35.

Es hat sich gezeigt, daß die Anwesenheit von Propylenglykol-Glucosid im Reaktionsendprodukt von Vorteil sein kann, so daß man nach einer bevorzugten Ausführungsform das Herstellungsverfahren so lenkt, daß maximal 15 Gew.-% Propylenglykol-Glucosid im Reaktionsgemisch verbleiben. Dabei kann der Restgehalt an Propylenglykol-Glucosid durch vorzeitigen Abbruch der Umacetalisierung bzw. durch bestimmte Variationen der Desillatmenge bei der Entfernung des überschüssigen Propylenglykols beeinflußt werden. Durch die Anwesenheit des Propylenglykol-Glucosids wird nicht nur die Fließfähigkeit des Reaktionsgemisches verbessert, wodurch die Abreicherung an Fettalkohol erleichtert wird, sondern auch die Hellfarbigkeit und Alkalistabilität des Endproduktes, so daß eine Bleichung des Endproduktes nicht mehr zwingend erforderlich ist.

Gegenstand der vorliegenden Erfindung sind auch bestimmte Alkylglucosid-Gemische als neue Erzeugnisse, wie sie nach dem hier beschriebenen und beanspruchten Verfahren erhalten werden können. Dabei enthält eine bevorzugte Ausführungsform weniger als 3 Gew.-% Restfettalkohol. Eine weitere bevorzugte Ausführungsform beinhaltet 50 bis 65 Gew.-% Alkylmonoglucosid, 8 bis 15 Gew.-% Alkyldiglucosid, 2 bis 5 Gew.-% Alkyltriglucosid, maximal 3 Gew.-% Restfettalkohol, 5 bis 15 Gew.-% Propylenglykol-Glucosid15 Gew.-% Propylenglykol-Glucosid und 5 bis 20 Gew.-% Polyglucose. In allen Erzeugnissen ist der Gehalt an freier Glucose vorzugsweise kleiner als 1 Gew.-%.

## BEISPIELE

Beispiel 1

Kartoffelstärke wurde im erfindungsgemäßen Einstufenverfahren zu einem oberflächenaktive Alkylglucosidverbindungen enthaltenden Reaktionsprodukt verarbeitet. Dabei kamen die folgenden Einsatzstoffe in den nachfolgend angegebenen Mengen zur Verwendung:

| | |
|---|---|
| 5,77 kg | Kartoffelstärke (Wassergehalt 18 %), entsprechend 4,73 kg Stärke (wasserfrei) und 1,04 kg Wasser |
| 13,80 kg | Propylenglykol, davon wurden 8,13 kg vorgelegt und 5,67 kg im Gemisch mit der Stärke zugegeben |
| 35 kg | $C_{12}/C_{14}$-Fettalkohol (native Basis; Gemisch aus ca. 75 Gew.-% Dodecanol und ca. 25 Gew.-% Tetradecanol) |
| 128 g | Paratoluolsulfonsäure-Monohydrat |
| 134 g | Magnesiumethylat zur Neutralisation |

Propylenglykol und $C_{12}/C_{14}$-Fettalkohol wurden zusammen mit dem Katalysator vorgelegt und auf ca. 120 °C erwärmt. Bei Erreichen der Temperatur begann entweder die kontinuierliche Zugabe des Slurry bestehend aus Propylenglykol und Kartoffelstärke oder die portionsweise Dosierung der Slurry in 3 Portionen. Dabei erfolgte die Zugabe der nächsten Portion, sobald eine klare Reaktionsmischung vorlag. Die Zugabe war nach ca. 50 Minuten beendet. Um die Stärkefeuchte zu entfernen, wurde unmittelbar nach der letzten Zugabe der Druck reduziert (100 mm Hg). Es destillierten 1,7 kg Flüssigkeit (Wasser und Propylenglykol) ab.

Durch stufenweises Reduzieren des Druckes (100/75/50/20/15 mm Hg) bei 115 bis 120 °C wurden weitere 11 kg Flüssigkeit (93 Gew.-% Propylenglykol/7 Gew.-% $C_{12}/C_{14}$-Fettalkohol) abdestilliert. Dabei erwies es sich als zweckmäßig, den aufsteigenden "Kühler" auf 80 °C zu erwärmen. Die Reaktionsmischung wurde eine halbe Stunde bei 80 °C mit Magnesiumethylat neutralisiert und auf einen pH-Wert zwischen 9 und 10 eingestellt. Anschließend wurde ungelöstes Magnesiumethylat (Rückstand 100 g) über einen 80 μm Filtersack abfiltriert.

Die Abdestillation des überschüssigen Fettalkohols erfolgte im Dünnschichtverdampfer im Vakuum bei ca. 1 Torr und einer Sumpftemperatur von maximal 160 °C.

Nach der Belüftung mit Stickstoff wurde eine Probe der Schmelze für analytische Bestimmungen gezogen. Das derart synthetisierte $C_{12}/C_{14}$-Glucosid bestand im wesentlichen aus 56 Gew.-% Monoglucosid, 12 Gew.-% Diglucosid, 3 Gew.-% Triglucosid, 12 Gew.-% Polyglucose, 5 Gew.-% Propylenglykolglucosid und 3 Gew.-% Restfettalkohol. Der Anteil an freier Glucose war kleiner als 1 Gew.-%. Die OH-Zahl betrug 652. Nach Abkühlung der Schmelze auf 100 °C wurde diese mit 5,5 kg Wasser, das auf 70 bis 80 °C vorgewärmt war, versetzt und gleichzeitig mit 0,5 % Wasserstoffperoxid (bezogen auf Aktivsubstanz) bei pH 10 (Zusatz von Natronlauge) 1 Stunde lang gebleicht.

Das auf diese Art erhaltene Produkt besaß eine Klett-Zahl von 10 (nach dem Farbstabilitätstest: 15). Eine vergleichbare Qualität (Klett-Zahl 31,7) wies ohne angeschlossene Bleiche lediglich ein Produkt auf, bei dessen Herstellung nur 83 % des Propylenglykols abdestilliert wurden (siehe Tabelle 1).

Beispiel 2

Kartoffelstärke wurde im erfindungsgemäßen Mehrstufenverfahren zu einem oberflächenaktive Alkylglucosidverbindungen enthaltenden Reaktionsprodukt verarbeitet. Dabei kamen die folgenden Einsatzstoffe in den nachfolgend angegebenen Mengen zur Verwendung:

| | |
|---|---|
| 5,77 kg | Kartoffelstärke (Wassergehalt 18 %), entsprechend 4,73 kg Stärke (wasserfrei) und 1,04 kg Wasser |
| 27,55 kg | Propylenglykol, davon wurden 8,13 kg vorgelegt, 5,67 kg im Gemisch mit der Stärke und 13,75 kg zusammen mit dem Fettalkohol zugegeben |
| 35 kg | $C_{12}/C_{14}$-Fettalkohol (native Basis; Gemisch aus 75 Gew.-% Dodecanol und 25 Gew.-% Tetradecanol) |
| 128 g | Paratoluolsulfonsäure-Monohydrat |
| 134 g | Magnesiumethylat zur Neutralisation |

Propylenglykol und der Katalysator wurden gemeinsam vorgelegt und auf ca. 120 °C erwärmt. Die Zugabe der Slurry aus Propylenglykol und Stärke erfolgte wie in Beispiel 1. Nach der Entfernung der Stärkefeuchte (Destillatmenge: 1,7 kg Flüssigkeit aus Wasser und Propylenglykol) wurde die auf 90 bis 100 °C vorgewärmte Mischung aus Propylenglykol und Fettalkohol im Vakuum bei 100 mm Hg eingetragen.

Die destillative Abreicherung an Propylenglykol sowie die Aufarbeitung des Produktgemisches erfolgten analog zu Beispiel 1.

Zusammensetzung und Qualität des Reaktionsproduktes wichen nicht wesentlich von den Daten des nach Beispiel 1 erhaltenen Produktes ab.

In weiteren Versuchen wurde eine vollständige Umacetalisierung durchgeführt (0 Gew.-% Propylenglykol-Glucosid im Reaktionsendprodukt).

Bei der Verwendung von MgO als Neutralisationsmittel wurde mit angeschlossener Bleiche ebenfalls ein Produkt von hoher Farbqualität (Klett-Zahl: 15, nach dem Farbstabilitätstest: 20) erhalten (s. Tabelle 1).

Tabelle 1

| Bestimmung der Klett-Zahlen von $C_{12}/C_{14}$-Glucosid (5 %ige Lösungen in $H_2O$: Isopropanol = 70 : 25) | | | | | |
|---|---|---|---|---|---|
| Neutralisationsmittel | Bleiche ja/nein | Gew.-% Propylenglykol-Glucosid | Bemerkung | Klett-Zahl | Farbe der Paste |
| Mg(OEt)$_2$ | + | 5 | - | 10 | hellgelb |
| Mg(OEt)$_2$ | + | 5 | pH 12**, 1 Stunde, 100 °C | 15 | hellgelb |
| Mg(OEt)$_2$ | - | 15 | 83 % PG* abdestilliert | 31,7 | hellgelb |
| MgO | + | - | - | 15 | hellgelb |
| MgO | + | - | pH 12**, 1 Stunde, 100 °C | 20 | hellgelb |
| NaOH/MgSO$_4$ | + | - | - | 27 | hellgelb |
| NaOH/MgSO$_4$ | + | - | pH 12**, 1 Stunde, 100 °C | 51,5 | dunkelgelb |
| NaOH | + | - | - | 50 | dunkelgelb |

* PG = Propylenglykol
** Farbstabilitätstest

## Ansprüche

1. Verfahren zur Herstellung von Alkylglucosiden aus einer Saccharid-Komponente und längerkettigen monofunktionellen Alkoholen mit im wesentlichen $C_8$- bis $C_{18}$- Alkyl- bzw. Alkenyl-Ketten nach der Umacetalisierungsmethode mit Propylenglykol, dadurch gekennzeichnet, daß zu einem auf 100 bis 130 °C erwärmten Reaktionsmedium (A), das mindestens aus Propylenglykol und einem sauren Katalysator und gegebenenfalls höherem Fettalkohol besteht, eine Saccharid-Komponente (B), die Stärke oder partielle Stärkeabbauprodukte enthält, zudosiert wird, wobei der eingesetzte Alkohol eine primäre OH-Gruppe besitzt und das molare Verhältnis von höherem Alkohol zu Propylenglykol maximal 1 ist.

6

2. Verfahren nach Anspruch 1, dadurch gekennzeichnet, daß das Reaktionsmedium (A) Propylenglykol in Mengen von 3 bis 8 Mol, bezogen auf 1 Mol Saccharid (berechnet als Anhydroglucose), enthält und höherer auf 90 bis 100 °C vorgewärmter Fettalkohol in Mengen von 3 bis 8 Mol pro Mol Anhydroglucose bei einem auf 100 mm Hg reduzierten Druck zu einem Gemisch aus (A) und (B) zudosiert wird, wobei gleichzeitig Wasser und Propylenglykol destillativ entfernt werden.

3. Verfahren nach Anspruch 1, dadurch gekennzeichnet, daß das Reaktionsmedium (A) ein Gemisch aus Propylenglykol in Mengen von 3 bis 8 Mol, bezogen auf 1 Mol Saccharid (berechnet als Anhydroglucose), und höherem Fettalkohol in Mengen von 3 bis 8 Mol pro Mol Anhydroglucose enthält und nach der Zugabe von (B) Wasser und Propylenglykol durch stufenweise Reduktion des Druckes im Bereich von 100 bis 15 mm Hg bei einer Temperatur von 115 bis 120 °C destillativ entfernt werden.

4. Verfahren nach einem der Ansprüche 1 bis 3, dadurch gekennzeichnet, daß die Saccharid-Komponente (B) aus einem Gemisch aus Stärke oder partiellen Stärkeabbauprodukten und Propylenglykol, insbesondere in Mengen von 2 bis 4 Mol Propylenglykol, bezogen auf 1 Mol Anhydroglucose, besteht.

5. Verfahren nach einem der Ansprüche 1 bis 4, dadurch gekennzeichnet, daß die Zugabe der Saccharid-Komponente (B) kontinuierlich erfolgt.

6. Verfahren nach Anspruch 1 oder 2, dadurch gekennzeichnet, daß der längerkettige monofunktionelle Alkohol im Gemisch mit Propylenglykol, vorzugsweise 0,5 bis 1,2 Mol Propylenglykol pro Mol eingesetztem höherem Fettalkohol, zu einer Mischung aus (A) und (B) hinzugegeben wird.

7. Verfahren nach einem der Ansprüche 1 bis 6, dadurch gekennzeichnet, daß zum Zwecke des Erhalts von Produkten mit hohem Farbqualität die Umacetalisierungsreaktion vorzeitig abgebrochen wird.

8. Verfahren nach einem der Ansprüche 1 bis 7, dadurch gekennzeichnet, daß nach der Umacetalisierung

a) der saure Katalysator mit einer organischen oder anorganischen basischen Alkali- oder insbesondere Erdalkaliverbindung neutralisiert und darüber hinaus auf einen pH-Wert von wenigstens 8, vorzugsweise 9 bis 10 eingestellt wird und

b) nach Filtration der überschüssige Fettalkohol auf eine übliche, das Reaktionsprodukt schonende Weise auf einen Wert von unterhalb 5 Gew.-% abdestilliert wird.

9. Verfahren nach einem der Ansprüche 1 bis 8, dadurch gekennzeichnet, daß der Katalysator eine Säure aus der Gruppe bestehend aus Schwefel-, Phosphor-, Paratoluolsulfonsäure, vorzugsweise Paratoluolsulfonsäure, ist und in einer Menge von 0,01 bis 0,03 Mol pro Mol der im eingesetzten Saccharid enthaltenen Glucose-Einheit verwendet wird.

10. Erzeugnis erhältlich nach dem Verfahren nach einem der Ansprüche 1 bis 9, dadurch gekennzeichnet, daß es im wesentlichen aus 50 bis 65 Gew.-% Alkylmonoglucosid, 8 bis 15 Gew.-% Alkyldiglucosid, 2 bis 5 Gew.-% Alkyltriglucosid, 5 bis 15 Gew.-% Propylenglykol-Glucosid, 5 bis 20 Gew.-% Polyglucose und maximal 3 Gew.-% Restfettalkohol besteht und der Gehalt an freier Glucose kleiner als 1 Gew.-% ist.

**Europäisches Patentamt**

# EUROPÄISCHER RECHERCHENBERICHT

Nummer der Anmeldung

EP 89 12 3750

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-2 036 472 (ATLAS) <br> * Insgesamt * & US-A-3 772 269 (Kat. D) <br> --- | 1-10 | C 07 H 15/04 |
| A | EP-A-0 032 252 (BASF) <br> * Patentanspruch * <br> --- | 1 | |
| A,D | EP-A-0 099 183 (STALEY) <br> * Patentansprüche 1-4 * <br> --- | 1 | |
| A,D | EP-A-0 102 558 (BASF) <br> * Patentansprüche 1-4 * <br> ----- | 1 | |

**RECHERCHIERTE SACHGEBIETE (Int. Cl.5)**

C 07 H 15/00

Der vorliegende Recherchenbericht wurde für alle Patentansprüche erstellt

| Recherchenort | Abschlußdatum der Recherche | Prüfer |
|---|---|---|
| DEN HAAG | 23-03-1990 | BRENNAN J. |